# EUROPÄISCHE PATENTSCHRIFT

(11) **EP 0 444 197 B1**
(45) Veröffentlichungstag und Bekanntmachung des Hinweises auf die Patenterteilung: **03.08.1994**
(21) Anmeldenummer: 90905372.0
(22) Anmeldetag: 15.09.1989
(51) Int. Cl.: A61F 2/30, A61F 2/36

(54) **ENDOPROTHESE DES HÜFTGELENKS**
ENDOPROSTHESIS OF THE HIP JOINT
ENDOPROTHESE DE L'ARTICULATION DE LA HANCHE

(43) Veröffentlichungstag der Anmeldung: 04.09.1991
(73) Patentinhaber: NAUCHNO-PROIZVODSTVENNOE OBIEDINENIE KOMPLEXNOGO RAZVITIA MEDITSINSKOI TEKHNIKI I IZDELY MEDITSINSKOGO NAZNACHENIA "EKRAN", Moscow, 129301 (RU)
(72) Erfinder: BELYKH, Sergei Ivanovich, Moscow, 129041 (SU); DAVYDOV, Anatoly Borisovich, Moscow, 111395 (SU); PETRULIS, Algimantas Juozovich, Kaunas, 233005 (SU); PRANTSKYAVICHUS, Sigitas Vladovich, Kaunas, 233043 (SU)
(74) Vertreter: Zellentin, Rüdiger
(86) Internationale Anmeldenummer: SU8900243
(87) Internationale Veröffentlichungsnummer: WO9103991

(56) Entgegenhaltungen:
- EP-A- 0 040 884
- EP-A- 0 196 258
- WO-A-87/04916
- WO-A-88/01492
- DE-A- 3 627 097
- FR-A- 2 315 902
- GB-A- 2 054 383
- US-A- 4 718 916
- US-A-47 189 09

## Beschreibung

Die Erfindung betrifft eine Hüftgelenk-Endoprothese mit einem in die Gelenkpfanne des Hüftbeins einführbaren Kopf und mit einem den Kopf tragenden, in den Markkanal des Oberschenkelknochens einsteckbaren Schaft.

Aus der US-A-3 982 281 ist eine Hüftgelenk-Endoprothese aus Metall bekannt, deren Kopf in einer im Hüftbein aufgenommenen Metallschale sitzt und deren Schaft in den Markkanal des Oberschenkelknochens eingeführt ist. Der Querschnitt des Schafts verringert sich von seinem kopfseitigen Ende zu seinem gegenüberliegenden Ende hin schrittweise, wobei auf seiner ganzen Länge seine maximale Breite stets kleiner als die zugeordnete Querabmessung des Markkanals ist. Zur Fixierung des Schaftes im Markkanal, aus dem das Mark und die schwammige Knochenschicht entfernt worden sind, wird Zement eingeführt, der dann aushärtet. Es hat sich gezeigt, daß diese Festlegung des Schaftes nicht zuverlässig ist und zu einer Lockerung der Endoprothese wie zu Auflösungen von Knochengewebe in den Wandbereichen führt. Die Endoprothese muß in der Regel nach fünf bis sieben Jahren ersetzt werden.

Bei einer Hüftgelenk-Endoprothese, wie sie aus der US-A-4 430 761 bekannt ist, hat der Schaft Keilform mit zwei parallelen ebenen Flächen, auf denen in Längsrichtung über ihrer ganzen Schaftlänge riefenförmige Vertiefungen ausgespart sind, die zu einer Vergrößerung der Haftfläche für den für die Fixierung verwendeten Zement dienen. Trotz dieser verbesserten Fixierung ergibt sich bei den zyklischen Beanspruchungen mit der Zeit aufgrund der großen Differenz zwischen dem Elastizitätsmodul des Zements und des Metalls des Schaftes eine Lockerung, was den Ersatz der Endoprothese nach fünf bis sieben Jahren bedingt.

Bei einer anderen bekannten Hüftgelenk-Endoprothese (I.A. Movshovich, Operativnaya ortopedia, 1983, Meditsina, Moskau, S. 203 - 207) besteht der Schaft aus einem Profilstab aus Metall, der in den freigelegten Markkanal paßdicht eingeführt wird. Obwohl dabei kein Zement verwendet wird, führt der ständige Vorspannungskontakt zwischen Profilstab und Knochengewebe mit der Zeit zu teilweiser Auflösung des Knochengewebes, wodurch der feste Sitz der Endoprothese verloren geht.

Wenn bei einem solchen Profilstab zusätzlich Knochenzement zur Fixierung verwendet wird (I.A. Movshovich, Operativnaya ortopedia, 1983, Meditsina, Moskau, S. 216 - 217), verteilen sich die Kraftbeanspruchungen über der Knochenoberfläche besser. Trotzdem stellt sich mit der Zeit eine Lockerung der Endoprothese aufgrund eines Abbaus von Knochengewebe in den Kontaktbereichen ein.

Die der Erfindung zugrunde liegende Aufgabe besteht nun darin, die Hüftgelenk-Endoprothese der eingangs genannten Art so auszugestalten, daß sie über einen langen Zeitraum einsatzfähig bleibt.

Diese Aufgabe wird ausgehend von der Hüftgelenk-Endoprothese der eingangs genannten Art dadurch gelöst, daß der Schaft in seinem kopfseitigen Abschnitt eine durchgehende Formausnehmung, an seinem gegenüberliegenden Endabschnitt eine Haube und an seinem dazwischenliegenden Abschnitt wenigstens zwei Längsnuten aufweist, daß durch die Formausnehmung eine U-förmige Klammer hindurchgeführt ist, die in der Formausnehmung durch einen Keil festklemmbar ist, und daß in jeder Längsnut eine verstellbare, aus einem am Schaft anliegenden Teil und einem am Markkanal anliegenden Teil zusammengesetzte Verbundeinlage aufgenommen ist, wobei die Klammer, der Keil und der am Marknagel anliegende Teil jeder Verbundeinlage aus einem biologisch abbaubaren Polymer, der am Schaft anliegende Teil jeder Verbundeinlage aus einem biologisch nicht oder langssam abbaubaren Polymer und die Haube aus einem biologisch nicht abbaubaren Polymer bestehen.

Durch die erfindungsgemäße Ausgestaltung der Hüftgelenk-Endoprothese ist es möglich, über lange Zeiträume eine Auflösung von Knochengewebe des Oberschenkelknochens im Bereich des Schaftes der Endoprothese zu vermeiden, wodurch die Einsatzdauer der Endoprothese mit unveränderter Funktion verlängert wird.

Die Länge jeder Längsnut soll das 0,7- bis 0,9-fache der Länge des Schaftes betragen, da bei einer größeren Länge die Anordnung der Haube nicht mehr möglich ist und bei einer geringeren Länge die Beweglichkeit der Verbundeinlage eingeschränkt wird, was die Fixierung der Endoprothese im Markkanal schwierig machen und verschlechtern würde.

Die Länge jeder Verbundeinlage sollte das 0,3- bis 0,6-fache der Länge des Schaftes betragen. Bei einer geringeren Länge des Schaftes ist die Einklemmfläche der Verbundeinlage nicht mehr ausreichend, was die Stabilität der Fixierung der Endoprothese beeinträchtigt. Ist die Länge der Verbundeinlage größer als das 0,6-fache der Schaftlänge, können die Teile der Verbundeinlage über die Längsnut vorstehen, was ihr späteres Entfernen erforderlich machen würde.

Das Einführen der Verbundeinlagen in den Markkanal wird dadurch begünstigt, daß die Form des am Schaft anliegenden Teils der Verbundeinlage der Form der Längsnut entspricht und der am Markkanal anliegende Teil der Verbundeinlage eine zylindrische oder konische Form hat. Diese Ausgestaltung sichert außerdem die maximal mögliche Kontaktoberfläche zwischen der Verbundeinlage und der Wand des Markkanals.

Um das Hineinwachsen von Knochengewebe in die Formausnehmung im Schaft zu ermöglichen, sollte eine minimale biologische Abbauzeit des Keils von mehr als zwei Monaten gegeben sein.

Zur Vermeidung postoperativer Komplikationen und zur Schaffung günstiger Bedingungen für die Regenerierung des Knochengewebes können die Verbundeinlagen, der Keil und die Klammer physiologisch aktive Arzneimittel oder Arzneimittelgemische enthalten.

Anhand von Zeichnungen werden Ausführungsbeispiele der Erfindung näher erläutert. Es zeigen
- Fig. 1: schematisch im Längsschnitt eine Hüftgelenk-Endoprothese nach der Erfindung,
- Fig. 2: den Schnitt II-II von Fig. 1,
- Fig. 3: in einer Ansicht wie Fig. 2 eine Modifizierung der Endoprothese,
- Fig. 4: im Längsschnitt eine Verbundeinlage mit einem zylindrischen markkanalseitigen Teil,
- Fig. 5: in einer Ansicht wie Fig. 4 eine Verbundeinlage mit einem konischen markkanalseitigen Teil,
- Fig. 6: in einer Einzelheit die Profilausbildung der Teile der Verbundeinlage gemäß Fig. 3 und
- Fig. 7: in einer Ansicht wie Fig. 6 eine weitere Profilausbildung der Teile der Verbundeinlage.

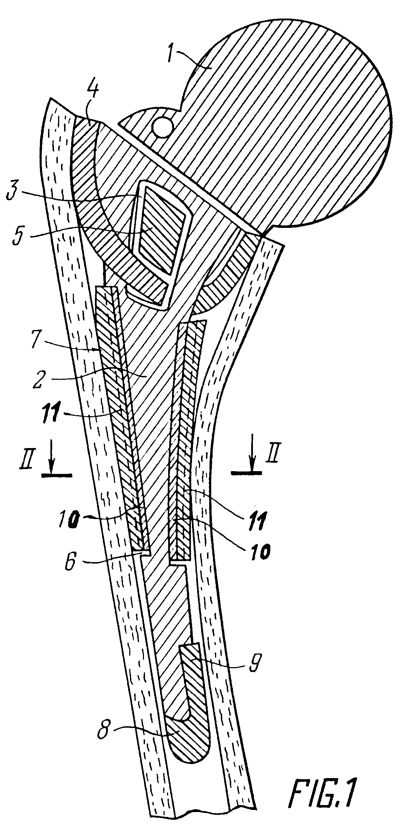

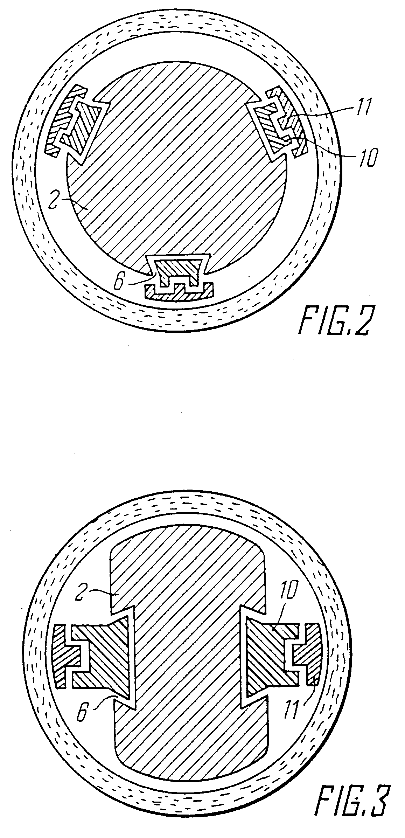

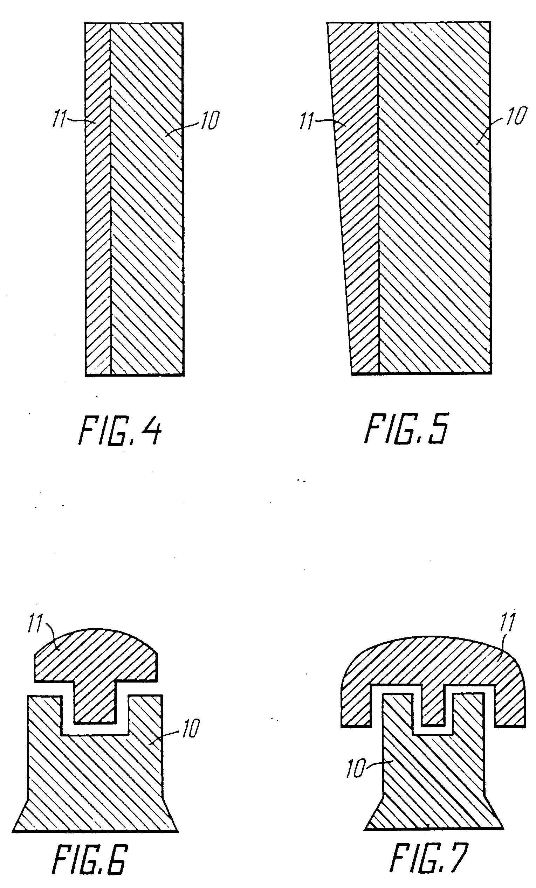

Die in Fig. 1 gezeigte Hüftgelenk-Endoprothese hat einen Kopf 1 und einen Schaft 2. Im kopfseitigen Abschnitt des Schaftes 2 ist eine durchgehende, als Profil ausgebildete Formausnehmung 3 angeordnet, durch die sich eine U-förmige Klammer 4 aus Polymer erstreckt, die in der Formausnehmung 3 durch einen Keil 5 aus Polymer fixiert ist. Im an den kopfseitigen Abschnitt anschließenden mittleren Abschnitt des Schaftes 2 sind Längsnuten 6 (Fig. 2 und 3) vorgesehen, in denen verstellbare Verbundeinlagen 7 aus Polymer angeordnet sind. Der dem kopfseitigen Abschnitt gegenüberliegende Endabschnitt des Schaftes 2 (Fig. 1) weist eine Haube 8 aus Polymer auf, die einen Vorsprung 9 hat.

Jede verstellbare Verbundeinlage 7 besteht aus zwei als Profile ausgebildeten Teilen 10 und 11 (Fig. 1, 2 und 3). Der in die Längsnut 6 des Schaftes 2 ragende Teil 10 entspricht der Form der Längsnut 6 und besteht aus einem bioinerten bzw. biologisch nicht abbaubaren oder aus einem langsam resorbierbaren Polymer. Der an der Wand des Markkanals anliegende Teil 11 besteht aus einem biologisch zersetzbaren Polymer und hat eine zylindrische oder konische Form (Fig. 4, 5, 6 und 7).

Der markkanalseitige Teil 11 der Verbundeinlage 7 bewirkt eine anfängliche zuverlässige Fixierung und ermöglicht nach seiner biologischen Zersetzung und Resorption die Regenerierung des Knochengewebes an dieser Stelle, was zu einer Verdickung des Knochens führt, wodurch die Langzeitfixierung der Endoprothese gewährleistet wird. Der schaftseitige Teil 10 der Verbundeinlage 7, die aus dem biologisch nicht oder sich langsam zersetzenden elastischen Verbundwerkstoff besteht, bewirkt nach dem Einwachsen der Endoprothese in das Knochengewebe eine Dämpfung, die auftretende ungleichmäßige zyklische Beanspruchungen ständig gleichmäßig verteilt, was eine Resorption von Knochengewebe ausschließt, wie sie sich bei direkter Berührung mit dem aus Metall bestehenden Schaft 2 der Endoprothese ergeben würde.

Jede im mittleren Teil des Schaftes ausgebildete Längsnut 6 hat eine Länge, die das 0,7- bis 0,9-fache der Länge des Schaftes 2 beträgt. Die von den profilförmigen Teilen 10 und 11 gebildete verstellbare Verbundeinlage 7, die in der zugeordneten Längsnut 6 aufgenommen ist, hat eine Länge, die dem 0,3- bis 0,6-fachen der Länge des Schaftes 2 entspricht.

Die U-förmige Klammer 4, die durch die durchgehende Formausnehmung 3 hindurchgeht, und der Keil 5, der die Klammer 4 in der Formausnehmung 3 festklemmt, bestehen aus biologisch zersetzbarem Polymer mit einer Zersetzungsdauer von mindestens zwei Monaten. Die Länge des Keils 5 soll kleiner als oder genauso groß wie der Durchmesser des Markkanals sein. Der Durchmesser bzw. die größte Abmessung des Querschnitts der Klammer 4 soll kleiner als oder genauso groß sein wie der Abstand zwischen dem Schaft 2 und der Wand des Markkanals.

Die am dem kopfseitigen Abschnitt gegenüberliegenden Endabschnitt des Schaftes 2 angebrachte Haube 8 besteht aus bioinertem bzw. biologisch nicht zersetzbarem Polymer. Sie sorgt dafür, daß eine Berührung des Schaftes 2 mit dem Knochengewebe vermieden wird und daß Spannungen an der Kontaktstelle reduziert werden.

Die Hüftgelenk-Endoprothese wird so ausgewählt, daß die Größe ihres Schaftes 2 etwas kleiner als der Durchmesser des Markkanals in seinem engsten Querschnitt ist. Die verstellbaren Verbundeinlagen 7 werden in die Längsnuten 6 eingesetzt. Dann wird die U-förmige Klammer 4 in die Formausnehmung 3 eingeführt und durch den Keil 5 befestigt. Daraufhin wird der Schaft 2 der Endoprothese in den Markkanal bis zur erforderlichen Tiefe eingeführt. Dabei werden die Enden der U-förmigen Klammer 4 gegeneinander bewegt und stehen mit der Wand des Markkanals in seiner breiten intertrochantären Zone in Berührung. Mit Hilfe eines Impaktors werden die verstellbaren Verbundeinlagen 7 in Distalrichtung bis zur ihrer steifen Verklemmung zwischen der inneren Cortikalschicht und dem Schaft 2 der Endoprothese bewegt. Die über den Markkanal hinausragenden Teile der Klammer 4 werden abgeschnitten, bevor der Kopf 1 der Endoprothese in die Hüftgelenkpfanne eingeführt wird.

### Beispiel 1

Die Hüftgelenk-Endoprothese hat einen Kopf 1 und einen Schaft 2 mit einem Querschnitt von 11 mm x 5 mm, einer Länge von 125 mm und mit zwei trapezförmigen Längsnuten 6 mit einer Breite von 8 mm und einer Länge von 110 mm, in denen zwei verstellbare Verbundeinlagen 7 mit einer Länge von 75 mm fixiert sind, deren schaftseitiger Teil 10 aus Polydodekanamid, hergestellt für medizinische Zwecke, und deren markkanalseitiger Teil 11 aus einem Copolymer aus N-Vinylpyrrolidon und Methylmethakrylat, verstärkt mit Kapronfaser, besteht.

An der inneren Oberfläche der Verbundeinlage 7 ist ein Film des Copolymers aus N-Vinylpyrrolidon und Methylmethakrylat aufgetragen, das 20 % Dioxiden oder Gentamyzin als antimikrobielles Präparat enthält. In der Formausnehmung 3 im kopfseitigen Abschnitt des Schaftes 2 ist durch den Keil 5, der aus einer Mischung aus Äthylzyanakrylat, Kalziumglukose, Orothsäure und Dioxidin besteht, die Klammer 4 aus einem Copolymer aus N-Vinylpyrrolidon und Tetramethylakrylat, verstärkt mit Kapronfaser, befestigt, die an den mit der Wand des Markkanals und mit dem Schaft 2 nicht in Berührung kommenden Oberflächen einen Überzug aus einem Gemisch eines Copolymers aus N-Vinylpyrrolidon und Butylmethakrylat und von Gentamyzin trägt. Die Klammer 4 hat eine Breite, die der Breite des Schaftes 2 entspricht, eine Stärke von 2 mm und eine Länge von 60 mm.

Der Schaft 2 hat an seinem Ende eine profilförmige Haube 8 aus Polydodekanamid, deren Außenfläche mit einem Film eines Copolymers aus N-Vinylpyrrolidon und Methylmethakrylat überzogen ist, das ein Gemisch aus Kaliumglukonat und Gentamyzin enthält.

Der Schaft 2 wird in den Markkanal eingeführt, dessen Breite im engen Teil gleich 13 mm ist. Mit Hilfe eines Impaktors werden die verstellbaren Verbundeinlagen 7 bis zum Festklemmen eingeführt. Bei der Einführung wird die Klammer 4 zusammengedrückt, wobei sie mit der Wand des Markkanals in Berührung kommt. Die jeweils 15 mm herausragenden Teile der Klammer 4 werden abgeschnitten.

Nach zwei Wochen erfolgt eine teilweise Beanspruchung des Hüftgelenks. Eine volle Beanspruchung erfolgt nach drei Monaten. Bei einer Prüfung nach einem Jahr wird die Induration des Knochengewebes an der Stelle der Einführung der gleitenden Verbundeinlagen 7 sowie das Auftreten von Schwammknochengewebe in dem gespreizten Teil an der Stelle der Anbringung der Klammer 4 nachgewiesen. Eine Prüfung nach drei Jahren ergibt eine einwandfreie Funktion des Hüftgelenks. Röntgenologisch wird das vollständige Fehlen einer Resorption von Knochengewebe nachgewiesen.

### Beispiel 2

Die Hüftgelenk-Endoprothese wird mit ihrem Schaft 2, der einen Querschnitt von 11 mm x 5 mm und eine Länge von 90 mm sowie Längsnuten 6 mit einer Breite von 7 mm und einer Länge von 75 mm aufweist, in das proximale Ende des Oberschenkelknochens eingeführt, dessen Markkanal in seinem engen Teil einen Durchmesser von 14 mm hat. In den Längsnuten 6 sind verstellbare Verbundeinlagen 7 mit einer Länge von 40 mm festgelegt, deren schaftseitiger Teil 10 aus einem Copolymer von N-Vinylpyrrolidon und Methylmethakrylat (Stickstoffgehalt 1,4 Gewichts%), verstärkt mit Lavsanfaser, besteht, und deren marknagelseitiger Teil 11 aus einem Copolymer aus N-Vinylpyrrolidon und Methylmethakrylat, verstärkt mit Kapronfaster (mit Säure behandelt) besteht.

Die schaftseitige Oberfläche jeder Verbundeinlage 7 ist mit einem Film aus dem gleichen Copolymer überzogen, das 15 % Karbonyzilin und 10 % Kaliumorothat enthält.

In der Formausnehmung 3 im kopfseitigen Abschnitt des Schaftes 2 ist durch den Keil 5 aus einem Copolymer aus N-Vinylpyrrolidon und Butylmethakrylat, das 30 % Kalziumglukonat enthält, und der eine Stärke von 11 mm hat, die Klammer 4 mit U-förmigem Querschnitt und einer Stärke von 2,5 mm befestigt, die aus dem Gemisch desselben Copolymers mit Polyalkylzyanakrylat, verstärkt mit Kapronfaser, ausgeführt ist. Die Länge der Enden der Klammer 4 beträgt 50 mm, ihr gegenseitiger Abstand im kopfseitigen Abschnitt 18 mm. Am Ende des Schaftes 2 ist die Haube 8 mit einer Länge von 12 mm aus einem Copolymer aus N-Vinylpyrrolidon und Methylmethakrylat (Stickstoffgehalt 1,4 Gewichts%) befestigt. Die Haube 8 hat an ihrem kopfseitigen Teil an der Stelle der Längsnut 6 einen Profilausschnitt. Wenn der Schaft 2 mit den Verbundeinlagen 7, die in seinem oberen Teil befestigt sind, bis auf die erforderliche Tiefe eingeführt wird, treten auch die Enden des Keils 5 in den Markkanal ein, wobei sie mit der Wand seines sich erweiternden Teils in Berührung kommen. Dann werden die Verbundeinlagen 7 zur Verspreizung eingetrieben, um die Endoprothese zu fixieren. Dabei kommt das durch die Haube 8 geschützte Ende der Endoprothese mit der Knochenwand in Berührung.

Nach zwei Wochen erfolgt eine teilweise Beanspruchung der Extremitäten. Eine vollständige Beanspruchung erfolgt nach sechsundneunzig Tagen. Bei einer Prüfung nach zwei Jahren wird eine stabile Lage der Endoprothese festgestellt. Röntgenologisch werden ein vollständiges Fehlen einer Resorption und eine Verdickung des Knochens an der Stelle der Einführung der Verbundeinlagen 7 und der Enden der Klammer 4 beobachtet.

### Beispiel 3

An dem proximalen Ende des Oberschenkelknochens mit einem Durchmesser im engen Teil von 13 mm wird der Schaft 2 der Endoprothese eingeführt, der einen Durchmesser von 9 mm und eine Länge von 90 mm sowie drei trapezförmige Längsnuten 6 mit einer Breite von 6 mm und einer Länge von 63 mm aufweist. In den Längsnuten 6 sind verstellbare Verbundeinlagen 7 mit einer Länge von 28 mm und einer Höhe des herausragenden Teils von 2 bis 2,5 mm befestigt. Der schaftseitige Teil 10 jeder Verbundeinlage 7 besteht aus Polypropylen, der marknagelseitige Teil 11 aus einem Copolymer aus N-Vinylpyrrolidon und Methylmethakrylat, verstärkt mit einer Faser aus Oxyzellulose. In der kopfseitigen Formausnehmung 3 des Schaftes 2 ist durch den Keil 5, der aus einem Gemisch aus Zyanakrylat und Knochenspänen besteht und eine Stärke von 13 mm hat, die Klammer 4 mit einem quadratischen Querschnitt mit einer Fläche von 4 cm² befestigt, die aus einem Copolymer aus N-Vinylpyrrolidon und Butylmethakrylat, verstärkt mit Kapronfaser, besteht. Die Länge der Enden der Klammer 4 beträgt 40 mm, ihr Abstand im oberen Teil 16 mm. Am Ende des Schaftes 2 ist die Haube 8 aus Polydodekanamid mit einer Länge von 14 mm befestigt, die in ihrem oberen Teil einen Profilausschnitt mit einer Länge von 8 mm aufweist.

Der Schaft 2 mit den zeitweilig in der oberen Stellung befestigten Verbundeinlagen 7 wird in den Markkanal bis zur erforderlichen Tiefe eingeführt, wobei die Enden der Klammer 4 in den sich erweiternden Teil des Markkanals eintreten und mit seiner Wand in Berührung kommen. Dann werden die Verbundeinlagen 7 eingeführt, die durch Spreizung die Fixierung der Endoprothese bewirken.

Nach zwölf Tagen erfolgt die teilweise Beanspruchung der Extremität. Eine volle Beanspruchung erfolgt nach vierundsechzig Tagen. Bei einer Prüfung nach siebenundzwanzig Monaten ist die Lage der Endoprothese stabil. Es wird ein vollständiges Fehlen einer Resorption von Knochengewebe nachgewiesen. Das Knochengewebe ist an der ganzen Oberfläche der Endoprothese kompakt.

### Beispiel 4

An dem proximalen Ende des Oberschenkelknochens, dessen Markkanal einen Durchmesser im engen Teil von 12 mm hat, wird der Schaft 2 der Endoprothese eingeführt, der einen Durchmesser von 8 mm und eine Länge von 85 mm sowie drei trapezförmige Längsnuten 6 mit einer Breite von 5 mm und einer Länge von 58 mm aufweist. In den Längsnuten 6 sind Verbundeinlagen 7 mit einer Länge von 27 mm und einer Höhe des herausragenden Teils von 1,9 mm bis 2,2 mm befestigt. Der schaftseitige Teil 10 jeder Verbundeinlage 7 besteht aus einem Copolymer aus N-Vinylpyrrolidon und Methylmethakrylat (Stickstoffgehalt 1,2 Gewichts%), der markkanalseitige Teil 11 aus dem gleichen Copolymer, das mit Säure behandelter Kapronfaser verstärkt ist. An den inneren Oberflächen der Verbundeinlagen 7 ist ein Überzug aus demselben Copolymer mit einem Gemisch aus Gentamyzin und Kaliumorothat vorgesehen. In der durchgehenden Formausnehmung 3 im kopfseitigen Abschnitt des Schaftes 2 ist mit Hilfe des Keils 5 aus polyäthylzyanakrylat, das ein Gemisch aus Kalziumglukonat, Orothsäure und Gluksidindioxidin enthält, die Klammer 4 mit einem runden Querschnitt von 2,2 mm Durchmesser befestigt, die aus einem Copolymer aus N-Vinylpyrrolidon und Methylmethakrylat, verstärkt mit Kapronfaser, besteht. Am Ende des Schaftes 2 ist die Haube 8 mit einem inneren Ausschnitt mit einer Länge von 6 mm befestigt. Die Endoprothese wird wie in Beispiel 3 beschrieben gesetzt.

Nach vierzehn Tagen erfolgt eine teilweise Beanspruchung der Extremität. Die vollständige Beanspruchung erfolgt nach 78 Tagen. Bei einer Prüfung nach zweiunddreißig Monaten ist die Lage der Endoprothese stabil. Eine Resorption von Knochengewebe fehlt vollständig. Es wird eine Verdickung des Knochens an der Stelle der Einführung der Verbundeinlagen 7 und im oberen Teil des Oberschenkelknochens beobachtet.

## Patentansprüche

1. Hüftgelenk-Endoprothese
- mit einem in die Gelenkpfanne des Hüftbeins einführbaren Kopf (1) und
- mit einem den Kopf (1) tragenden, in den Markkanal des Oberschenkelknochens einsteckbaren Schaft (2),
**dadurch gekennzeichnet,**
- daß der Schaft (2) in seinem kopfseitigen Abschnitt eine durchgehende Formausnehmung (3), an seinem gegenüberliegenden Endabschnitt eine Haube (8) und an seinem dazwischenliegenden Abschnitt wenigstens zwei Längsnuten (6) aufweist,
- daß durch die Formausnehmung (3) eine U-förmige Klammer (4) hindurchgeführt ist, die in der Formausnehmung (3) durch einen Keil (5) festklemmbar ist, und
- daß in jeder Längsnut (6) eine verstellbare, aus einem am Schaft (2) anliegenden Teil (10) und einem am Markkanal anliegenden Teil (11) zusammengesetzte Verbundeinlage (7) aufgenommen ist,
- wobei die Klammer (4), der Keil (5) und der am Markkanal anliegende Teil (10) jeder Verbundeinlage (7) aus einem biologisch abbaubaren Polymer,
- der am Schaft (2) anliegende Teil (11) jeder Verbundeinlage (7) aus einem biologisch nicht oder langsam abbaubaren Polymer und
- die Haube (8) aus einem biologisch nicht abbaubaren Polymer bestehen.

2. Hüftgelenk-Endoprothese nach Anspruch 1, **dadurch gekennzeichnet,** daß die Länge jeder Längsnut (6) das 0,7- bis 0,9-fache der Länge des Schafts (2) beträgt.

3. Hüftgelenk-Endoprothese nach Anspruch 1 oder 2, **dadurch gekennzeichnet,** daß die Länge jeder Verbundeinlage (7) das 0,3- bis 0,6-fache der Länge des Schaftes (2) beträgt.

4. Hüftgelenk-Endoprothese nach einem der Ansprüche 1 bis 3, **dadurch gekennzeichnet,** daß die Form des am Schaft (2) anliegenden Teils (10) jeder Verbundeinlage (7) der Form der Längsnut (6) entspricht und daß der am Markkanal anliegende Teil (11) der Verbundeinlage (7) eine zylindrische oder konische Form hat.

5. Hüftgelenk-Endoprothese nach einem der vorhergehenden Ansprüche, **dadurch gekennzeichnet,** daß die biologische Abbauzeit des Keils (5) mehr als zwei Monate beträgt.

6. Hüftgelenk-Endoprothese nach einem der vorhergehenden Ansprüche, **dadurch gekennzeichnet,** daß die Verbundeinlagen (7), der Keil (5) und die Klammer (4) physiologisch aktive Arzneimittel und/oder Arzneimittelgemische enthalten.

## Claims

1. A hip-joint endoprosthesis
- comprising a head (1) which can be introduced into the joint socket of the hip-bone and
- comprising a shaft (2) which carries the head (1) and which can be inserted into the medullary canal of the thigh-bone,
characterized
- in that the shaft (2) has in its head-end portion a continuous shaped recess (3), at its opposite end portion a cap (8) and on its intervening portion at least two longitudinal grooves (6),
- in that a U-shaped clip (4) is passed through the shaped recess (3), which clip can be secured firmly in the shaped recess (3) by a wedge (5), and
- in that accommodated in each longitudinal groove (6) is an adjustable bonding insert (7) composed of a part (10) adjoining the shaft (2) and a part (11) adjoining the medullary canal,
- wherein the clip (4), the wedge (5) and the part (10) adjoining the medullary canal of each bonding insert (7) are made of a biologically degradable polymer,
- the part (11) adjoining the shaft (2) of each bonding insert (7) is made of a biologically non- or slowly degradable polymer, and
- the cap (8) is made of a biologically non-degradable polymer.

2. A hip-joint endoprosthesis according to claim 1, characterized in that the length of each longitudinal groove (6) is 0.7 to 0.9 times the length of the shaft (2).

3. A hip-joint endoprosthesis according to claim 1 or 2, characterized in that the length of each bonding insert (7) is 0.3 to 0.6 times the length of the shaft (2).

4. A hip-joint endoprosthesis according to one of claims 1 to 3, characterized in that the shape of the part (10) of each bonding insert (7) adjoining the shaft (2) corresponds to the shape of the longitudinal groove (6) and in that the part (11) of the bonding insert (7) adjoining the medullary canal has a cylindrical or conical shape.

5. A hip-joint endoprosthesis according to one of the preceding claims, characterized in that the biological degradation time of the wedge (5) is more than two months.

6. A hip-joint endoprosthesis according to one of the preceding claims, characterized in that the bonding inserts (7), the wedge (5) and the clip (4) contain physiologically active drugs and/or drug mixtures.

## Revendications

1. Endoprothèse de l'articulation de la hanche, comportant :
- une tête (1) pouvant être insérée dans la cavité glénoïde de l'os iliaque et
- une tige (2) portant la tête (1) et pouvant être enfichée dans le canal médullaire du fémur, caractérisée en ce que :
- la tige (2) comporte un évidement continu (3) dans son segment de tête, une calotte (8) dans son segment terminal opposé et au moins deux rainures longitudinales (6) dans son segment intermédiaire,
- une agrafe en U (4) est passée par l'évidement (3), laquelle peut être fixée dans l'évidement (3) par une clavette (5) et
- chaque rainure longitudinale (6) loge une pièce intercalaire (7), réglable, constituée d'une partie (10) s'appuyant contre la tige (2) et d'une partie (11) s'appuyant contre le canal médullaire,
- l'agrafe (4), la clavette (5) et la partie (10), s'appuyant contre le canal médullaire, de chaque pièce intercalaire (7), étant en un polymère biodégradable,
- la partie (11), s'appuyant contre la tige (2) de chaque pièce intercalaire (7), étant constituée d'un polymère non-biodégradable ou lentement biodégradable, et
- la calotte (8) étant constituée d'un polymère non-biodégradable.

2. Endoprothèse de l'articulation de la hanche selon la revendication 1, caractérisée en ce que la longueur de chaque rainure longitudinale (6) est de 0,7 à 0,9 fois égale à la longueur de la tige (2).

3. Endoprothèse de l'articulation de la hanche selon la revendication 1 ou 2, caractérisée en ce que la longueur de chaque pièce intercalaire (7) est de 0,3 à 0,6 fois égale à la longueur de la tige (2).

4. Endoprothèse de l'articulation de la hanche selon l'une des revendications 1 à 3, caractérisée en ce que la forme de la partie (10) s'appuyant contre la tige (2), de chaque pièce intercalaire (7), correspond à la forme de la rainure longitudinale (6), et en ce que la partie (11) de la pièce intercalaire (7) s'appuyant contre le canal médullaire, a une forme cylindrique ou conique.

5. Endoprothèse de l'articulation de la hanche selon l'une des revendications précédentes, caractérisée en ce que le temps de dégradation biologique de la clavette (5) est supérieur à deux mois.

6. Endoprothèse de l'articulation de la hanche selon l'une des revendications précédentes, caractérisée en ce que les pièces intercalaires (7), la clavette (5) et l'agrafe (4) contiennent des médicaments et/ou des mélanges de médicaments physiologiquement actifs.
